# EUROPEAN PATENT APPLICATION

(11) **EP 3 351 304 A1**
(43) Date of publication of application: **25.07.2018**
(21) Application number: 17152887.0
(22) Date of filing: 24.01.2017
(51) Int. Cl.: B01L 3/00, G01N 33/487, G01N 21/84, G01N 21/78, G06T 7/00, G01N 21/27, G01N 35/00, A61B 10/00

(54) **TEST CUP FOR ANALYSING A FLUID SPECIMEN, DEVICE FOR ANALYSING A FLUID SPECIMEN,KIT OF PARTS, AND METHOD**

(71) Applicant: Meseg, Thomas, 13125 Berlin (DE)
(72) Inventor: Meseg, Thomas, 13125 Berlin (DE)
(74) Representative: Zimmermann & Partner Patentanwälte mbB

(57) **Abstract**

A test cup (1) for analysing a fluid specimen includes a bottom (2), a wall (3) extending from the bottom (2) and an opening (5) opposite the bottom (2), the bottom (2) and the wall (3) defining a cavity with an inner side (6) for accommodating a fluid specimen containing an analyte. A test region (10) is arranged on or at the bottom (2) and contains a binding partner for the analyte, wherein a reaction of the binding partner with the analyte causes a change of a colour intensity of at least a portion of the test region (10) when the test region (10) comes in contact with the analyte of the fluid specimen. At least one of a colour calibration reference element (12) for a colour based calibration and a location reference element (11) for a location calibration, is arranged on or at the bottom (2). The test region (10) and at least one of the location reference element (11) and the colour calibration reference element (12) are arranged on or at the bottom (2) such that they are in the same field of view (102) of a camera (101), when the camera (101) is placed in a distance to the bottom (2) and is facing the bottom (2).

## Description

Embodiments described herein relate to a test cup and particularly to a disposable test cup which can be used to analyse a fluid specimen. Further embodiments pertain to a device for analysing a fluid specimen. Further embodiments relate to kit of parts and methods for analysing a fluid specimen.

### BACKGROUND

Immunoassays such as lateral flow rapid tests are widely used in in-vitro diagnostics, veterinary medicine and environmental analytics. These tests are based on an interaction of antibodies with antigens which brings about a change of a colour, or of colour or chromatic intensity or saturation of a test result strip. The change of the colour or of the colour saturation can be assessed either with the naked eye or quantitatively using an optical reader. There are known stationary devices and portable devices for evaluating such tests.

For example, US 2012/0063652 A1 describes a smartphone-based method for evaluating colour-based reaction testing of biological materials by capturing a digital image of a test strip together with an adjacently-located reference colour chart.

US 7 803 322 B2 describes a test media reader module.

WO 2009/054729 A1 describes an immunoassay analysis method.

The above described approaches, however, do not allow a cheap and reliable arrangement for analysing the digital image of a test strip. Image configurations for example brightness, contrast and white balance depend on the used camera and light source. In view of the above, there is need for improvement.

### SUMMARY

According to an embodiment, a test cup for analysing a fluid specimen, particularly a disposable test cup, includes a bottom, a wall extending from the bottom and an opening opposite the bottom. The bottom and the wall defining a cavity having an inner side for accommodating a fluid specimen containing an analyte. The test cup includes a test region arranged on or at the bottom and containing a binding partner for the analyte. A reaction of the binding partner with the analyte causes a change of a colour intensity, or of a colour, of at least a portion of the test region when the test region comes in contact with the analyte of the fluid specimen. The test cup has at least one of a colour calibration reference element for a colour based calibration and a location reference element for a location calibration, arranged on or at the bottom, wherein the test region and at least one of the location reference element and the colour calibration reference element are arranged such that they are in the same field of view of a camera, when the camera is placed in a distance to the bottom and is facing the bottom.

According to an embodiment, a device for analysing a fluid specimen, includes a test cup, particularly a test cup as described herein, and a test cup holder for holding the test cup and for accommodating the camera, wherein the test cup holder is adapted to hold the test cup in a distance to the camera facing the bottom of the test cup, wherein the test region and at least one of the location reference element and the colour calibration reference element are arranged such that they are in the same field of view of the camera.

According to an embodiment, a kit of parts includes a test cup, particularly a test cup as described herein, a manual and optionally a packing for the test cup.

According to an embodiment, a method for analysing a fluid specimen includes: providing a test cup; filling at least a portion of the cavity of the test cup with a fluid specimen, optionally removing the fluid specimen after a given time; processing the image, wherein processing includes at least one of locating the test region in the image, and calibrating colour values of the image, and analysing the test region in the image for detecting the presence and/or the concentration of the analyte in the fluid specimen.

Those skilled in the art will recognize additional features and advantages upon reading the following detailed description, and upon viewing the accompanying drawings.

### DETAILED DESCRIPTION

In the following Detailed Description, reference is made to the accompanying drawings, which form a part hereof, and in which are shown by way of illustration specific embodiments in which the invention may be practised. In this regard, directional terminology, such as "top", "bottom", "front", "back", leading", "trailing", vertical", "lateral" etc., is used with reference to the orientation of the Figure(s) being described. Because components of embodiments can be positioned in a number of different orientations, the directional terminology is used for purpose of illustration and is in no way limiting. It is to be understood that other embodiments may be utilised and structural or logical changes may be made. The following detailed description, therefore, is not to be taken in a limiting sense, and the scope of the present invention is defined by the appended claims. The embodiments being described use specific language, which should not be construed as limiting the scope of the appended claims.

According to an embodiment, a test cup for analysing a fluid specimen, particularly a disposable test cup, has a bottom, a wall extending from the bottom and an opening opposite the bottom. The bottom and the wall defines a cavity with an inner side for accommodating a fluid specimen containing an analyte. The test cup includes a test region arranged on or at the bottom and containing a binding partner for the analyte, wherein a reaction of the binding partner with the analyte causes a change of a colour intensity of at least a portion of the test region when the test region comes in contact with the analyte of the fluid specimen. The test cup includes at least one of a colour calibration reference element for a colour based calibration and a location reference element for a location calibration, arranged on or at the bottom, wherein the test region and at least one of the location reference element and the colour calibration reference element are arranged such that they are in the same field of view of a camera, when the camera is placed in a distance to the bottom and is facing the bottom. Typically, the colour calibration reference element and/or the location reference element is an element separate to the test region. The respective reference element or reference elements are arranged in close proximity to the test region and is or are typically not identical with or part of the test region.

The cavity of the test cup can be formed and defined by a bottom and a wall which extends from the bottom to an opening. The opening opposite the bottom defines an entrance into the cavity. The cavity can be filled with a fluid through the opening. The cavity can accommodate a fluid specimen and is sufficiently impermeable for aqueous liquids for an appropriate time to allow performing the analysis.

The inner side of the cavity can be defined by portions of the bottom and the wall. The bottom and the wall have respective inner sides facing and delimiting the cavity.

According to an embodiment, the bottom and the wall can include several layers or sections. The layers or sections may provide different functions. For example, one layer may provide the test cup with the impermeability for aqueous liquids and another layer may provide the test cup with a sufficient structural stability.

According to an embodiment, the wall can be formed to have a cylindrical shape or a partially cylindrical shape. For example, the wall can be formed such that it has a circular or elliptical footprint on the bottom. Alternatively or additionally, the wall can be formed as a prism or any other suitable shape. The shape of the inner side of the wall defines the shape of the cavity. The wall can also be provided to form a conus with increasing width towards the opening.

In an embodiment, the bottom of the test cup has a circular shape. The wall runs along the periphery of the bottom and substantially extends vertical relative to the bottom. The wall defining a curved lateral surface with increasing diameter, at least partially, from the bottom to the opening. The opening may be formed by a bare end of the wall, or by a structural element which can be formed by an upper portion of the wall, or by a separate element attached to the wall. The structural element can be, for example, a back-folded or rolled end portion of the wall to increase the dimensional stability of the wall at the opening.

According to an embodiment, the bottom and wall of the test cup are made of mainly inexpensive material for example coated paper or plastic. Thus, the manufacturing costs for test cup can be reduced which allows providing the test cup as a disposable product.

The opening is located opposite the bottom. According to an embodiment, the area defined by the opening can be parallel to the bottom. Alternatively, the area of the opening is not parallel to the bottom. The opening can be, for example, defined by the end of the wall or by a rim which is attached to the wall. According to an embodiment, the opening is wide enough to allow fluid to be poured in without additional means like a pipette. This is beneficial for patients and medical personal who collects samples such as urine samples.

The test region can be arranged on or at the bottom and typically contains a binding partner for an analyte. Chemical (analytical) tests like, urine test strips, are well known diagnostic tools to determine pathological parameters or identify diseases. The test region may have one, two or a plurality of analytic areas or test pads each selective for a given analyte. Each analytic area or test pad contains a specific binding partner for a chemical reaction with a corresponding analyte. For a chemical reaction of the binding partner and the corresponding analyte, the test region is brought into contact with a fluid that contains the analyte or the analytes. The analytic area or test pad may get in full contact or in partial contact with the fluid. Chemical reaction includes formation of covalent bonds, ionic bonds or of any other bonds that results in a colour or colour intensity change of the respective test pad.

According to an embodiment, the test region has a first test pad with a binding partner for a first analyte and a second test pad with another binding partner for a second analyte contained in the fluid specimen, and a reaction of one of the binding partners with the corresponding analyte causes a change of a colour intensity of at least a portion of the corresponding test pad when the test pad comes in contact with the analyte in the fluid specimen.

According to an embodiment, the test region has a plurality of different test pads. Each test pad contains a specific binding partner for a chemical reaction with an analyte. Colour changes of the test pads can be of different colours or colour intensities. The test pads can be arranged in the test region in any order such as a row, a matrix or in an irregular order.

According to an embodiment the test region includes a substrate. The binding partner of the analyte can be bound to the substrate in selective areas of the test region. The substrate can be, for example, a tissue to which the binding partner is covalently bound or otherwise immobilized. The one or more test pads can be formed by one or more integral parts of the substrate, or can be formed by an additional pad material, which contains the binding partner, attached to the substrate. The test pad can therefore also be referred to as analytic area of the substrate.

According to an embodiment, the chemical reaction between the analyte and the binding partner causes a change of the colour or colour intensity of the test pad, which may be visible for the human eye and can be analysed in a digital image of the test region, or selected test pads, captured by a camera. The colour change can be a change of colour intensity or of colour type (hue). The change of the colour or colour intensity may not be directly visible for naked human eye but can be made visible using the camera which can be susceptible to and can record infrared light and ultraviolet light.

The colour type or the colour intensity can be used for a qualitative analysis, a semi-quantitative or even a quantitative analysis. The qualitative analysis merely determines the presence or absence of the analyte. Semi-quantitative analysis provides a result related to a scale, which gives an estimation of a quantitative result. The semi-quantitative analysis can be done, if the change of colour intensity is approximately proportional relative to the concentration of the analyte in the specimen. Quantitative analysis can be done, if the colour change is directly related to the concentration of the analyte in the specimen.

According to an embodiment, the chemical reaction causes a colour change of the one or more test pads, which can be used for a quantitative analyse of the concentration of the analyte and the fluid specimen.

According to an embodiment, the test cup can be used for analysing any fluid specimen, which contains an analyte corresponding to a binding partner of the test region.

According to an embodiment, the test region has a blind test region and/or a reference region. Using a blind test and or the reference test is very common in such test strips and can prevent the test results from being false positive or false negative.

The chemical reaction of the analyte and its binding partner is according to an embodiment irreversible. Therefore the test region can only be used once. If the test region is permanently attached to the test cup, the test cup can only be used once. The test region can be made of inexpensive, disposable material, which are made for a single use. The test region typically does not include electronic evaluation devices. The evaluation is done by a human or by a software which analyses an images of the test region captured by a digital camera.

The test cup includes at least one of a colour calibration reference element for a colour based calibration and a location reference element for a location calibration, arranged on or at the bottom. It can be either one of them or both. They are arranged on or at the bottom together with the test region such that they are in the same field of view of a camera, when the camera is placed in a distance to the bottom and is facing the bottom. The camera can therefore take an image of these elements.

According to an embodiment, the colour calibration reference element is used for a colour based calibration in a digital image. The colour calibration reference element is adapted to the colour change of the test region. It can be used for example to adjust at least one of brightness, contrast, and white balance. The colour and the colour change of the test region, particularly of the test pad or pads, may change depending on the actual camera used. To provide a comparable and uniform, or standardized, interpretation of the colour and the colour change irrespective of the used digital camera, the colour calibration reference element is provided and an image thereof is captured together with an image of the test region. The test region and the calibration reference are contained in the same image so that images of both are captured under identical conditions such as illumination conditions.

According to an embodiment, the test cup has a location reference element for location calibration arranged on or at the bottom. The test region and the location reference elements are arranged such that they are in the same field of view of a camera, when the camera is placed in a distance to the bottom and is facing the bottom. The location reference element provides a means for locating the test region in the image captured by a camera, when the camera is placed in a distance to the bottom and is facing the bottom. The location and the distortion of the test region depend on, for example the camera optics, the camera angle and the focus settings.

According to an embodiment, the location reference element and the colour calibration reference element are formed as one structural element which provides the functionality of for locating and colour calibration. For example, colour bars or dots of different colour (hue), intensity (saturation) and lightness (brightness) can be arranged in a given geometrical relation. The arrangement of the colour bars or dots can be used as location reference.

According to an embodiment, the location reference element can also be configured to be sufficiently distinctive relative to its immediate surrounding so that the location reference element is suitable to support focussing of the camera, when the camera is placed in the distance to the bottom and is facing the bottom.

According to an embodiment, the distance between the opening and the bottom (i.e. the height) is at least 60 mm, typically at least 80 mm. According to an embodiment, the distance between the opening and the bottom is from about 60 mm to about 140 mm, particularly from about 80 mm to about 120 mm. The test cup may have a given minimum size if the camera is placed direct on the opening to take the image of the test region. The given minimum size may be defined by the minimum focus distance of common cameras.

According to an embodiment, the size of the bottom, or the diameter of the cup at the bottom, is between about 60 mm to about 100 mm, particularly between 60 mm to about 80 mm.

The size of the test cup can be adapted to facilitate collecting of the specimen and handling of the test cup so that the patient or the medical personnel does not need further means such as pipettes or cones to fill fluid specimen into the test cup.

According to an embodiment, the aspect ratio of the height of test cup from the bottom to the opening of the test cup to the diameter of the test cup at the bottom is between at least 60:100 and 140:60, particularly between 100:100 and 140:80. A test cup having an aspect ratio within the above range is provided with a wall that is sufficiently large to ensure that the fluid specimen can be easily collected and is not spilled during use.

Typically, the volume of the cavity, as defined by the bottom and wall of the test cup, can be between about 0.1 l and 0.5 l to facilitate collection and handling of the fluid specimen. The volume of the fluid specimen is typically much lower than the total volume of the cavity. For analysis purposes, only a small amount of fluid specimen is typically needed to cover the bottom.

According to an embodiment, at least a portion of the test region and at least one of the location reference element and the colour calibration reference element are arranged at the inner side of the cavity, i.e. within the cavity, such that they are in the same field of view of a camera, particularly of a camera of a mobile electronic device, when the camera facing the bottom is placed on or above the opening of the test cup.

According to another embodiment, at least a portion of the test region and at least one of the location reference element and the colour calibration reference element are arranged on or at the bottom at an outer side of the cavity such that they are in the same field of view of a camera, when the camera facing the bottom is placed at the outer side of the cavity.

The test region and the location reference element and/or the colour calibration reference element can either be arranged on or at a side of the bottom which faces the inside of the cavity, or can be arranged on or at an opposite side of the bottom which faces away from the cavity and to the outside of the cavity. The side of the bottom which faces the inside of the cavity is also referred to as inner side of the bottom. The opposite side of the bottom which faces away from the cavity and to the outside of the cavity is also referred to as outer side of the bottom. When arranged on the inner side of the bottom, the test region typically comes into direct contact with the liquid when the liquid is filled into the cavity. When arranged on the outer side of the bottom to face away from the cavity, the test region may not come directly in contact with the liquid filled into the cavity. In this case, a fluid guiding element may be provided to guide the fluid contained in the cavity to the test region.

According to an embodiment, the wall includes a rim defining the opening. The rim can be an integral part of the wall, or can be formed separately and attached to an upper end of the wall opposite the bottom.

Further according to an embodiment the test cup can include a lid releasable attached to the wall for closing the opening, particularly releasably attached to a rim of the wall, wherein the lid has a hole for a camera for a mobile electronic device when the mobile electronic device with its camera is placed on the lid. A lid can favourably cover the opening of the test cup to prevent ambient light from entering the cavity.

The camera may have its own light source to illuminate the test region and at least one of the location reference element and the colour calibration reference element. The lid can have a common hole for the camera and the light source. Alternatively, two separate holes can be provided in the lid; one for the camera and one for the light source.

According to an embodiment, the lid has a positioning element for positioning the mobile electronic device such that the camera is arranged on or above the hole. The positioning element can be helpful to arrange the camera of the mobile electronic device above the hole in an optimal position. The positioning element can include a limit stop arranged in a way that a camera has to be placed on the lid directly at the limit stop to get a predefined optimal point of view. The positioning element can be dependent on the geometry of the mobile electronic device and/or the camera.

According to an embodiment, the cup further includes a fluid guiding element to guide at least a portion of the fluid specimen from the inner side of the cavity to a contact area of the test region, when at least a portion of the cavity is filled with the fluid, particularly if the test region is arranged at an outer side of the cavity. The fluid guiding element connects the inner side of the cavity with at least a portion of the test region. A fluid in the cavity is guided to the test region to start the chemical reaction of the analyte and its binding partner.

According to an embodiment, the location reference element and/or the colour calibration reference element, and the test region are permanently attached on or at the bottom of the test cup and the test cup is a single-use test cup. Alternatively, the location reference element and/or the colour calibration reference element are permanently attached and the test region is non-permanently attached, for example replaceable. In a further embodiment, the location reference element and/or the colour calibration reference element, and the test region are permanently attached to a common holder which is placed on the bottom of the test cup. The common holder can be, for example, a carrier sheet material such as a plastic or a paperboard material onto which the location reference element and/or the colour calibration reference element, and the test region are permanently attached. The common holder can be placed with the side onto which the reference element and the test region is attached facing towards the opening of the test cup onto the bottom or can be fixed on the bottom. The common holder can be exchangeable, or can be adapted to be attachable shortly before use.

The test cup can be made for a single use only. The cost of the used material and the production is relatively cheap compared to analyses with laboratory equipment. The test cup can be used at home by a patient or laymen or medical or technical personnel in laboratories. This is beneficial as it significantly reduces the costs for the specimen analysis. Currently known devices are designed for multiple uses and thus need to be extensively cleaned to avoid cross-contaminations between different analyses. Hence, the reliability of the analysis is improved.

According to an embodiment, the test cup is a non-electronic device. That means that all parts of the test cup mentioned above are non-electronic devices and/or the test cup does not additionally includes or comprises any electronic devices.

According to an embodiment, the cavity is packaged and/or releasably closed. The cavity and/or the whole cup can be releasably closed. For example the cavity can be closed by a cover that creates an air-proof package. Alternatively or in addition, the whole cup is packed in a foil. The package can protect the inner side of the cavity and the test region of contamination and moister. Contamination or moisture can damage the test regions or can lead to incorrect test results. The package can be manufactured in a cost-efficient manner. For example, the opening of the test cup can be sealed by a plastic foil or an aluminium foil to create an air-proof package..

According to another embodiment, a kit of parts is provided. The kit of parts includes a test cup as described herein, a manual and optionally a packing for the test cup. If the test cup is used by a patient, a manual, which explains the use of the test cup, may be useful. The manual can be separate or can be for example printed on the package or on the test cup.

According to an embodiment, the bottom and the wall are non-transparent, particularly opaque. This may be useful if the test region and at least one of the location reference element and the colour calibration reference element are arranged at the inner side of the cavity. It can prevent ambient light from the outside of the cavity to enter the cavity. The quality of the digital image of the test region and at least one of the location reference element and the colour calibration reference element captured by the camera typically depends on the illumination. Irregular light conditions caused by ambient light from the outside of the test cup can be avoided with a non-transparent, particularly opaque or sufficiently opaque, bottom and wall so that the illumination conditions are mainly defined by the light source of the mobile device.

According to an embodiment, the test cup further includes a spacer arranged at an outer side opposite to the inner side of the bottom, wherein the spacer creates a space between the bottom and an area surface the test cup stands on. That spacer might be particularly useful if the test region and one of the colour calibration reference element and the location reference element are located at the outer side of the bottom. The spacer keeps the test region at a given distance from the window so that during handling the test region is protected against direct contact with any surface on which the test cup is placed.

The test cup can be placed on a transparent window and the camera can be placed under the test cup on the window to take the image. The spacer can be non-transparent, particularly opaque, to avoid distracting light and create a nearly standardized illumination while taking the image.

Further according to a developed embodiment, the spacer and the bottom are creating a second cavity accommodation the test region and at least one of the location reference element and the colour calibration reference element. This second cavity can be closed by a packing as well to protect the test region as describes above.

According to an embodiment, a device for analysing fluid specimen is provided. The device has a test cup and a test cup holder for holding the test cup and for accommodating the camera. The test cup holder is adapted to hold the test cup in a distance to the camera facing the bottom of the test cup, wherein the test region and at least one of the location reference element and the colour calibration reference element are arranged such that they are in the same field of view of the camera.

According to an embodiment, the holder has a window above the camera. The test cup can be placed, with its bottom, on one side of the window while the camera is arranged on the other side of the window with the field of view on the test region and at least one of the location reference element and the colour calibration reference element.

According to an embodiment, a method for analysing a fluid specimen by using the test cup is provided. The method includes: providing a test cup; filling at least a portion of the cavity of the test cup with a fluid specimen; capturing an image with a camera of the test region and at least one of the location reference element and the colour calibration reference element in one image; perform on the image at least one of locate the test region by use of the reference element, and calibrate the colour of the image by the use of the colour calibration reference.

The method can be used with any combinations of the embodiments of the test cup explained above. Adaptions of the method are related to the different embodiments of the test cup.

### BRIEF DESCRIPTION OF THE DRAWINGS

The components in the figures are not necessarily to scale, instead emphasis being placed upon illustrating the principles of the invention. Moreover, in the figures, like reference numerals designate corresponding parts. In the drawings:
Figures 1 illustrates a test cup according to an embodiment, a mobile device with a camera and an image made by the camera;
Figures 2A and 2B illustrate a test cup according to an embodiment with an additionally lid;
Figure 3 illustrates a test cup according to an embodiment;
Figures 4 illustrates a image made by the camera according to an embodiment;
Figure 5 illustrates a device for analysing a fluid specimen having a test cup and a holder;
Figure 6 illustrates a cross-section of the bottom with a test region arranged at the outer side of the bottom according to an embodiment;
Figure 7 illustrates a cross-section of the bottom with a test region arranged at the outer side of the bottom according to an embodiment; and
Figure 8 illustrates a cross-section of the bottom with a test region arranged at the outer side of the bottom according to an embodiment.

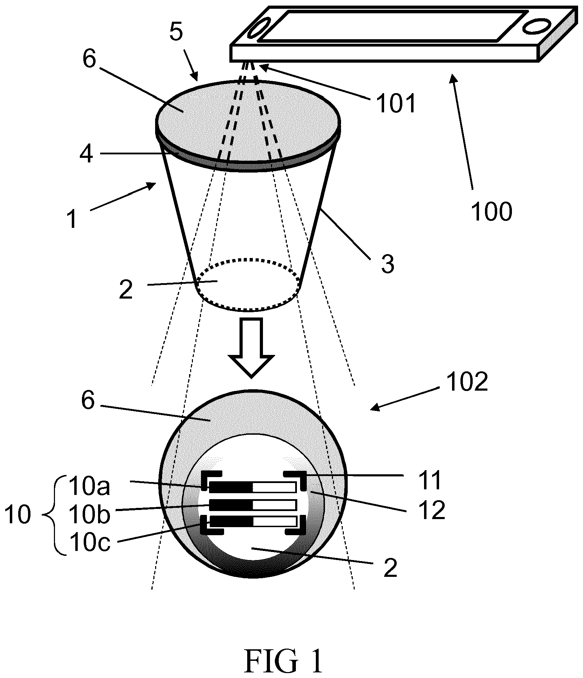

Figure 1A illustrates a test cup 1 according to an embodiment, a mobile electronic device 100 and an image 102 taken by a camera 101 of the mobile electronic device 100. The test cup 1 includes a bottom 2 and a wall 3 forming a cavity having an inner side 6 for a accommodating fluid specimen. A wall 3 is attached on one end to the bottom 2 and extends from the bottom 2 to an opening 5 which is arranged opposite the bottom 3. The wall 3 can have, for example as shown in Figure 1, a circular cross-section with an increasing diameter towards the opening 5. At the opening 5, the cup has a rim 4. The rim 4 is attached to the wall 3 and defines the border of the opening 6.

The wall 3 and the bottom 2 of the test cup 1 can be of the same or of different material. Typically, both the wall 3 and the bottom 2 are of the same material.

According to an embodiment, the material is a laminate, or a two ply board, having at least two layers of different material. One layer can be, for example, a stabile paperboard to provide structural stability. The other layer can be, for example, a liquid-tight foil laminated to the paperboard, typically to the side of the paperboard which is facing the cavity. The foil can be, for example, a plastic foil or an aluminium foil.

The material for the bottom and the wall is typically non-transparent, typically substantially light-tight to prevent ambient light to enter the cavity. A light-tight layer or ply may be additionally laminated to the paperboard if needed.

According to an embodiment, the material is a laminate, or a three ply board, having at least three layers of different material. In addition to an internal stable paperboard layer providing sufficient structural stability, respective outer layer are laminated onto the paperboard layer.

A plastic material may be used as alternative to the paperboard layer. Examples for suitable plastic materials are polyethylene (PE), polyvinylchloride (PVC), polyacrylate (PC), or polystyrene (PS).

As perhaps best shown in the lower illustration of Figure 1, which illustrates an image captured by a camera from above the opening 6, a test region 10 having at least one test pad 10a, 10b, 10c is arranged on an inner side of the bottom 2. Furthermore, location a reference element 11 and a colour calibration reference element 12 is arranged at the bottom 2. The test region 10 as well as the reference element 11 and the colour calibration reference element 12 are visible from the opening 6 and are not obstructed by any structural element.

The at least one test pad 10a, 10b, 10c contains at least one binding partner for a given analyte to be detected. The analyte can be, for example, a protein, a hormone, a metabolic product, or a drug without being limited thereto. For analysing the presence and/or the concentration of the analyte in a fluid specimen, the specimen is filled into the cavity so that the fluid specimen comes into contact with the test region 10 and at least with the test pad 10a, 10b, 10c.

The mobile electronic device 100 is then placed in or at a distance to the bottom 2 and is facing the bottom 2 to capture an image 102 of the test region 10 along with the reference element 11 and the colour calibration reference element 12. In the embodiment of Figure 1, the camera 101 is placed above the opening 5 to capture the image 102. The illustrated image 102 is the result of the point of view of the camera 101 in that arrangement.

The test region 10, particularly the at least one test pad 10a, 10b, 10c, containing the binding partner for the analyte is arranged at the inner side 6 of the cavity on the bottom 2, which is illustrated in the image 102 representing a plan view onto the inner side of the bottom 2. The location reference element 11 and the colour calibration reference element 12 are located at the inner site 6 of the cavity and arranged on the bottom 2.

The test region 10 is illustrated in the present embodiment to include three test pads 10a, 10a, 10c. Each test pad 10a, 10a, 10c includes a binding partner corresponding to a respective analyte contained in the specimen. The test pads 10a, 10a, 10c are formed as test strips. Each of the test pads 10a, 10a, 10c includes a probe area which changes its colour or colour intensity when a chemical reaction between the binding partner and the corresponding analyte occurs. In the present embodiment, each of the test pads 10a, 10a, 10c further includes a reference area which allows evaluation of the quality of the test pad or which provides a reference colour intensity. A comparison between the reference area and the probe area may allow a semi-quantitative analyse of the analyte content and/or the analyte concentration.

The location reference element 11 is arranged around the test region 10 as illustrated in the image 102. The location reference element 11 can be used a means to facilitate focussing of the camera 101 when capturing the image. Additionally, an image processing using a computer-aided analysis of the image 102 can be performed to allocate the test region 10 in the image. The location reference element 11, an image of which is contained in the image captured by the camera, facilitate the identification of the test region 10 within the image so that only the section of the captured image that corresponds to the test region needs to be analysed.

The colour calibration reference element 12 is arranged on the bottom 2 next to the test region 10. A general pre-calibration of a camera prior to capturing the image is generally not needed for the analyses because the illumination conditions may vary between individual images taken. When taking an image, the test region 10 and the colour calibration element 12 are typically illuminated by the same light and under comparable conditions because they are arranged close to each other. For improving control of the illumination conditions, the light source of the mobile device is used.

Typically, the mobile electronic device 100 includes a light source, the camera, a processing unit and a memory. An analysis software can be stored in the memory and executed for capturing the image and for analysing the image to obtain a test result that allows an assessment of the presence or absence of the analyte to be detected, and/or a quantitative evaluation of the content and/or concentration of the analyte in the fluid specimen.

After capturing the image, a software algorithm typically evaluates the test area 10 in the image 102 by identifying first the location reference element 11 and the colour calibration reference element 12. Alternative or additionally the image 102 and/or the evaluated results can be sent to a laboratory or medical experts to perform the evaluation and/or to make a diagnosis.

In addition to the location reference element 11 and the colour calibration reference element 12, a bar code or a QR-Code may be provided on the bottom 2 to be visible for the camera. The bar code or the QR code, or any other suitable machine-readable code, may store information relevant for evaluating the test region. For example, the code may identify the test region 10 and the type of analytes which can be detected and evaluated by the test region 10. Furthermore, the expiration date of the test region 10 can also be stored.

After capturing the image, the analysis software may also evaluate the code and can import data needed for the evaluation of the test region 10 either from an internal database or an external database.

The image analysis and processing to obtain a test results can be performed as described in US 2014/0286550 A1, the content of which is hereby incorporated by reference. Briefly, the process can include:
Step 01: Acquisition (capturing) of an image of the test region 11, the location reference element 11 and the colour calibration reference element 12.
Step 02 (Optional): Identification of test type (type of test region) from barcode or QR-code attached to or printed on the bottom of the test cup.
Step 03: Identification of the location reference element 11 for geometric calibration of features in the image.
Step 04 (Optional): Application of geometric image calibration, for example perspective transformation, to the image based on an analysis of the shape and location of the location reference element 11 in the captured image.
Step 05: Identification of the colour calibration reference element 12 for colorimetric calibration in designated calibration areas such as the test region 10.
Step 06: Storage of information for geometric and colorimetric calibration in database of the mobile device.
Step 07: Identification of all probe regions and reference regions of each test pad.
Step 08: Quantitative evaluation of the probe regions and the reference regions of the test pads by employing information for colorimetric calibration.
Step 09: Determination of test result (e.g. positive/negative/invalid) using test type specific algorithms and parameters (e.g. thresholds) employing an appropriately parameterized logical function.
Step 10 (Optional): Displaying test results.
Step 11 (Optional): Storage of test results in database on mobile device.
Step 12 (Optional): Transmission of test results to external database.

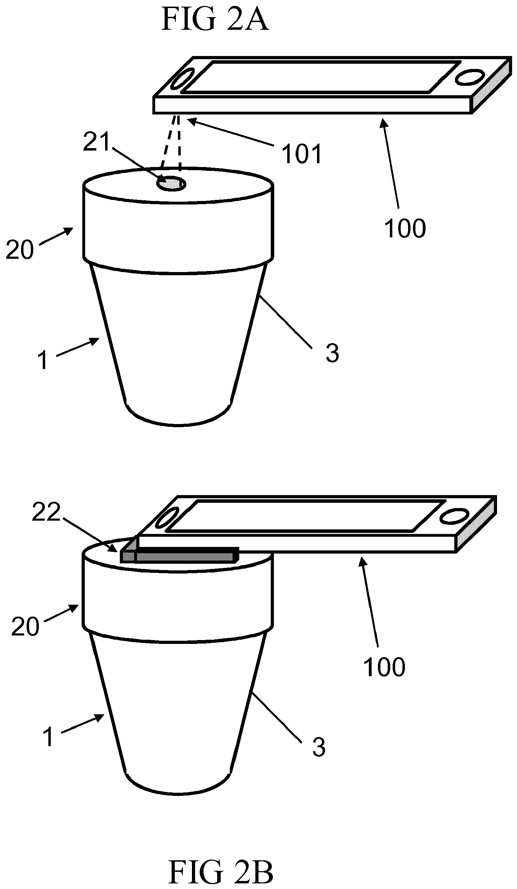

Figure 2A and 2B illustrate a test cup 1 according to an embodiment with an additionally lid 20. The test cup 1 can be basically identical to the test cup as illustrated in Figure 1.

In Figure 2A, the lid 20 is attached to the wall 3 and includes a hole or opening 21. The image 102 can be taken by the camera 101 through the hole 21. Typically, the camera 102 is placed very close to the hole 21 or directly on the lid 20. In Figure 2A, the distance from the camera 102 to the hole 21 may be too large to obtain an appropriate image 102. The illustrated arrangement was chosen to show all relevant parts.

In Figure 2B, the lid additionally includes a positioning element 22. The camera 101 is placed directly on the lid 20 above the hole 21, which is covered by the mobile device 100 and therefore not illustrated here. The mobile device is arranged by the positioning element in an optimal position to align the camera with the hole 21 so that the camera is directly above the hole 21. This improves the point of view for the camera to take the image 102.

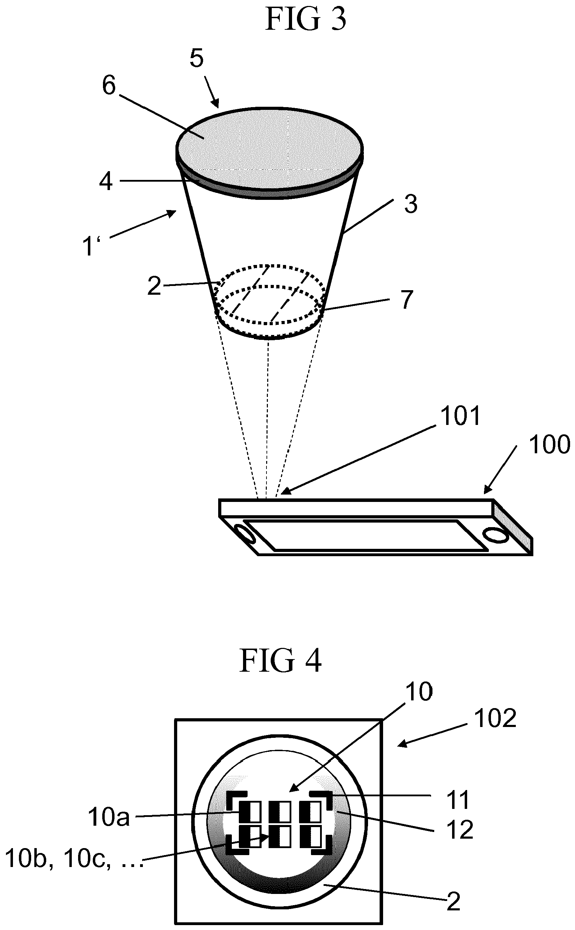

Figure 3 illustrates a test cup 1' according to another embodiment. Figure 4 illustrates an image taken by the mobile device 100 in the arrangement of Figure 3.

A difference between the test cup 1 according to the embodiment of Figures 1, 2A and 2B and the test cup 1' according to the embodiment of Figures 3 and 4 is the location of the test region 10, the location reference element 11 and the colour calibration reference element 12. These elements are arranged at the outer side of the cavity at an outer side of the bottom. The camera 101 takes the image 102 while is placed in a distance to the bottom 2 and is facing the outer side of the bottom 2.

When using the test cup 1 of the embodiments illustrated in Figure 1, 2A and 2B, the liquid specimen may be removed, for example poured out from the cavity, prior to capturing an image to avoid that the liquid specimen, which may be turbid or cloudy, affects the image capturing. If needed, the cavity may be quickly rinsed with water to remove excess liquid specimen from the test region 10.

Arranging the test region 10, the location reference element 11 and the colour calibration reference element 12 on or at the outer side of the bottom allows a direct view onto these elements without any interference with the liquid specimen that is contained in the cavity. It is therefore not needed to remove the liquid specimen from the cavity.

The test cup 1' can additionally include a spacer 7 arranged at an outer side opposite to the inner side of the bottom 2. The spacer 7 and the bottom 2 are forming a second cavity or a space. The test region 10, the location reference element 11 and the colour calibration reference element 12 are arranged in the second cavity.

The test cup 1' can further include a fluid guiding element to guide at least a portion of the fluid specimen from the inner side 6 of the cavity into a contact area of the test region 10, when at least a portion of the cavity is filled with the fluid.

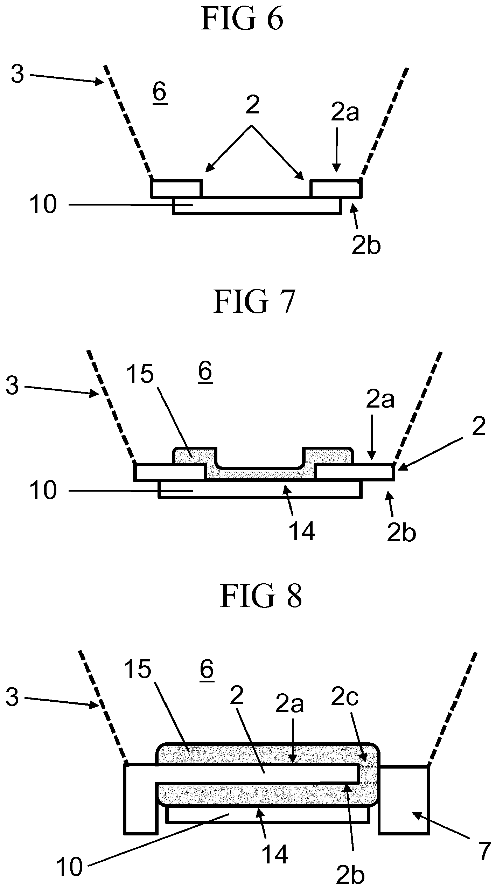

Figures 6 to 8 illustrates embodiments for arranging the test region 10, and the location reference element 11 and the colour calibration reference element 12 (not illustrated in Figures 6 to 8 for ease of illustration) at or on the second side 2b of the bottom 2. Reference sign 2a denotes the inner side of the bottom on which the test region 10, and the location reference element 11 and the colour calibration reference element 12 are arranged in the embodiments of Figures 1, 2A and 2B.

Figure 6 illustrates a portion of a test cup having a bottom with a central opening which is covered from the outer side 2b by the test region 10. The backside of the test region 10, which faces the opening in the bottom 2, comes into direct contact with the fluid specimen. The front side of the test region 10, i.e. the side that faces away from the opening in the bottom 2, bears the test pad or test pads.

Figure 7 illustrates a portion of a test cup having a bottom with a central opening which is covered from the outer side 2b by the test region 10. The central opening is also covered on the inner side 2a of the bottom by a fluid guiding element 15 which is in direct contact with the test region 10 in the opening of the bottom. The fluid guiding element 15 guides the fluid specimen to a contact area 14 of the test region 10. The contact area 14 is the interface between the fluid guiding element 15 and the test region 10.

Figure 8 illustrates a portion of a test cup having a bottom 2 with a small opening 2c through which the fluid guiding element 15 extends from the inner sider 2a to the outer side 2b of the bottom. The fluid guiding element 15 covers the inner side 2a of the bottom 2 and is covered at the outer side 2b by the test region 10.

Figure 8 also illustrates the spacer 7 which is arranged at the outer side of the bottom 2 to keep the outer side 2b of the bottom 2 and the test region 10 at a given distance from a surface when the test cup 1 is placed on the surface.

In addition to guiding fluid to the test region 10, the fluid guiding element 15 may also provide some filtration function to remove dirt or other unwanted components, which may affect the test results, from the test region 10. For example, the fluid guiding element 15 can provide some column functionality for component retention.

Figure 4 illustrates an image taken by the mobile device 100 in the arrangement of Figure 3, where the outer side 2b of the bottom 2 can be seen. The location reference element 11 and the colour calibration reference element 12 are similar to the elements of the embodiment illustrated in Figure 1. The test region 10 includes a plurality of test pads 10a, 10b, 10c. Each test pad contains a binding partner for an analyte in the fluid specimen and the binding partner of each pad 10a, 10b, 10c reacts with the respective analyte when coming in contact with fluid specimen. The intensity of the reaction, being a measure of the content or concentration of the respective analyte in the fluid specimen, is visible, and detectable, by an individual change of the colour or colour intensity of the respective test pad. The colour calibration reference element 12 is ideally adapted to provide a calibration for all possible colour changes of the test pads.

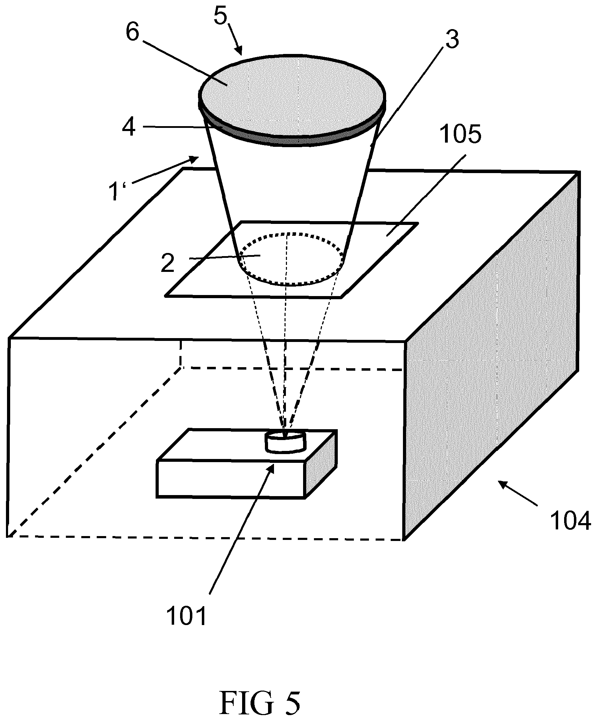

Figure 5 illustrates a device for analysing a fluid specimen having a test cup 1 and a holder 104. The test cup 1' is a test cup with test region 10, location reference element 11, and colour calibration reference element 12 arranged at the outer side 2b of the bottom similar to Figures 3 and 4. The test cup 1' is placed on a window 105 of the holder 104 for evaluating the test results.

An integrated camera 103 is arranged or placed under or inside the holder 104. The test cup 1' is arranged on the window 105 in a distance to the camera 103, which is facing the bottom of the test cup, wherein the test region 10 and at least one of the location reference 11 element and the colour calibration reference element 12 are arranged such that they are in the same field of view of the camera 103. The image is similar to the image illustrated in Figure 4.

### Reference list

US 2012/0063652 A1
WO 2013/1433533 A1
US2014/0154789 A1
US2014/0286550 A1
WO 2016/025332 A1
US 8 935 007 B2
US 2006/0222567 A1
US 8 145 431 B2
US 8 655 009 B2
EP 2 921 846 A2
US 2012/0282154 A1
EP 2 781 910 A1
US 7 803 322 B2
WO 2009/054729 A1
WO 00/46598 A1
US 5 902 982
US2003/0206829 A1
US 2005/0106750 A1
US 2006/0280650 A1
US 7 458 942 B2
US2004/0132091 A1
WO 92/17109 A1
WO 2010/075814 A1
US 5 429 804
EP 1 284 802 B1
US2014/0271367 A1
US 2014/0242612 A1
US2013/0162981 A1
US2013/0184188 A1
US 2005/0095697 A1
US 7 267 799 B1

## Claims

1. A test cup (1) for analysing a fluid specimen, particularly a disposable test cup (1), comprising:
- a bottom (2), a wall (3) extending from the bottom (2) and an opening (5) opposite the bottom (2), the bottom (2) and the wall (3) defining a cavity with an inner side (6) for accommodating a fluid specimen containing an analyte;
- a test region (10) arranged on or at the bottom (2) and comprising a binding partner for the analyte, wherein a reaction of the binding partner with the analyte causes a change of a colour intensity of at least a portion of the test region (10) when the test region (10) comes in contact with the analyte of the fluid specimen; and
- at least one of a colour calibration reference element (12) for a colour based calibration and a location reference element (11) for a location calibration, arranged on or at the bottom (2),
- wherein the test region (10) and at least one of the location reference element (11) and the colour calibration reference element (12) are arranged on or at the bottom (2) such that they are in the same field of view (102) of a camera (101), when the camera (101) is placed in a distance to the bottom (2) and is facing the bottom (2).

2. A test cup (1) according to claim 1, wherein at least a portion of the test region (10) and at least one of the location reference element (11) and the colour calibration reference element (12) are arranged on or at an inner side (2a) of the bottom (2) facing the inner side (6) of the cavity such that they are in the same field of view (102) of the camera (101), particularly of a camera of a mobile electronic device (100), when the camera (101) facing the bottom (2) is placed on or above the opening (5) of the test cup (1).

3. A test cup (1) according to any of the claims 1 or 2, further comprising a lid (20) releasably attached to the wall (3) for closing the opening (5), particularly releasably attached to a rim (4) of the wall (3), wherein the lid (20) comprises a hole (21) for the camera (101) of a mobile electronic device (100) when the mobile electronic device (100) with its camera (101) is placed on the lid (20).

4. A test cup (1) according to claim 3, wherein the lid (20) comprises a positioning element (22) for positioning the mobile electronic device (100) such that the camera (101) is aligned with and arranged on or above the hole (21).

5. A test cup (1) according to claim 1, wherein at least a portion of the test region (10a) and at least one of the location reference element (11) and the colour calibration reference element (12) are arranged on or at an outer side (2b) of the bottom (2) facing away from the inner side (6) of the cavity such that they are in the same field of view (102) of a camera (101), when the camera (101) facing the outer side of the bottom (2) is placed at a distance from the outer side (2b) of the bottom (2).

6. A test cup (1) according to any of the claims 1 to 5, wherein the cup further comprises a fluid guiding element (15) to guide at least a portion of the fluid specimen from the inner side of the cavity to a contact area (14) of the test region (10), when at least a portion of the cavity is filled with the fluid.

7. A test cup (1) according to any of the previous claims, wherein the location reference element (11), the colour calibration reference element (12), and the test region (10a) are permanently or non-permanently attached on or at the bottom (2) of the test cup (1), and wherein the test cup (1) is a single-use test cup.

8. A test cup (1) according to any of the previous claims, wherein the bottom (2) and the wall (3) are non-transparent, particularly opaque.

9. A test cup (1) according to any of the previous claims, the test region (10) comprises a first test pad (10a) with a first binding partner for a first analyte and a second test pad (10b) with a second binding partner for a second analyte contained in the fluid specimen, and a reaction of one of the first and second binding partners with the corresponding first and second analyte causes a change of a colour intensity of at least a portion of the corresponding test pad (10a, 10b) when the test pad (10a, 10b) comes in contact with the analyte in the fluid specimen.

10. A test cup according to any of the previous claims, wherein the cavity is packaged and/or releasably closed.

11. A test cup according to any of the previous claims, wherein the distance between the opening (5) and the bottom (2) is at least 60 mm, particularly at least 80 mm, and wherein optionally a diameter of the test cup at the bottom is at least 60 mm, particularly at least 80 mm.

12. A test cup according to any of the previous claims, wherein the test cup further comprises a spacer (7) arranged at the outer side of the bottom (2) to keep the outer side (2b) of the bottom (2) at a given distance from a surface when the test cup (1) is placed on the surface.

13. Device for analysing a fluid specimen, comprising:
- test cup (1) according to according to any of the claims 1 to 12, and
- a test cup holder (104) for holding the test cup and for accommodating the camera,
- wherein the test cup holder (104) is adapted to hold the test cup (1) in a distance to the camera facing the bottom (2) of the test cup (1), wherein the test region (10) and at least one of the location reference element (11) and the colour calibration reference element (12) are arranged such that they are in the same field of view of the camera.

14. Kit of parts, comprising a test cup (1) according to any of the previous claims, a manual and optionally a packing for the test cup (1).

15. Method for analysing a fluid specimen comprising:
- providing a test cup (1) according to any of the claims 1 to 11,
- filling at least a portion of the cavity of the test cup (1) with a fluid specimen containing at least one analyte to be detected, so that the fluid specimen comes into contact with the test region (10) of the test cup (1),
- capturing an image of the test region (10) and at least one of the location reference element (11) and the colour calibration reference element (12) with a camera,
- processing the image, wherein processing comprises at least one of locating the test region (10) in the image, and calibrating colour values of the image, and
- analysing the test region in the image for detecting the presence and/or the concentration of the analyte in the fluid specimen.
